Europäisches Patentamt

European Patent Office

Office européen des brevets

(11) Numéro de publication: **0 420 813 A1**

# (12) DEMANDE DE BREVET EUROPEEN

(21) Numéro de dépôt: 90810724.6

(22) Date de dépôt: **20.09.90**

(51) Int. Cl.5: **F16C 11/10, A61B 17/60**

(30) Priorité: 27.09.89 CH 3499/89

(43) Date de publication de la demande:
**03.04.91 Bulletin 91/14**

(84) Etats contractants désignés:
**AT BE DE DK ES FR GB GR IT LU NL SE**

(71) Demandeur: **JAQUET ORTHOPEDIE S.A.**
**5, chemin des Aulx**
**CH-1228 Plan-les-Ouates(CH)**

(72) Inventeur: **Wagenknecht, Marcel**
**Chemin des Milans 12**
**CH-1219 Le Lignon(CH)**

(74) Mandataire: **Dietlin, Henri**
**Dietlin & Cie S.A. Rue des Epinettes 19**
**CH-1227 Genève(CH)**

(54) Dispositif d'articulation et de blocage relatif de deux pièces.

(57) Ce dispositif d'articulation permet d'orienter une première pièce allongée 20 susceptible d'être pivotée de 360° selon la flèche A et une seconde pièce 30 apte à basculer sur l'arbre 40 selon la flèche B. Ces pièces sont rendues solidaires d'un support en U 10 par rapport auquel elles peuvent être bloquées au moyen de sabots 60 et 70 maintenus par une vis de serrage 50 unique.

Les pièces 20 et 30 peuvent être raccordées à des éléments devant être orientés, tels que des fiches ou des tiges de liaison faisant partie d'un fixateur externe orthopédique composé de fiches insérées dans des fragments osseux et d'une armature externe comportant des tiges de liaison.

Fig. 7

## DISPOSITIF D'ARTICULATION ET DE BLOCAGE RELATIF DE DEUX PIÈCES

La présente invention est du domaine de la mécanique et concerne un dispositif d'articulation entre deux pièces destinées à être bloquées dans une position spécifique, spécialement étudié pour être utilisé dans un fixateur externe chirurgical.

Lorsque deux pièces doivent être positionnées avec plus d'un degré de liberté angulaire, on utilise soit des systèmes permettant le réglage séparé selon plusieurs axes, soit des joints universels. Dans le premier cas, le blocage se fait par des systèmes de serrage en nombre correspondant à celui des axes de rotation, ce qui implique plusieurs opérations de serrage. Dans le second, on utilise des articulations à rotules pouvant être bloquées par une opération de serrage unique, mais qui présentent l'inconvénient de limiter les possibilités de positionnement en raison de la présence des mâchoires entourant la partie sphérique à maintenir.

La présente invention vise a pallier ces inconvénients et a en conséquence pour objet un dispositif d'articulation et de blocage relatif de deux pièces allongées, caractérisé par le fait qu'il comporte :

a) un support en U apte à recevoir lesdites pièces,

b) une première pièce pivotable sur son axe,

c) une seconde pièce basculable sur un arbre disposé perpendiculairement audit axe de la première pièce, et

d) des moyens de blocage disposés de part et d'autre desdites première et seconde pièces, sur un arbre de serrage unique.

Dans une forme d'exécution préférentielle, le support en U comporte une partie centrale, présentant un passage central pour la première pièce et deux ailes comportant des ouvertures situées sur un axe commun aptes à recevoir l'arbre de serrage.

Selon une première variante, l'arbre de serrage est disposé parallèlement aux ailes, tandis que dans une seconde variante il est disposé entre les ailes, perpendiculairement à celles-ci.

L'invention s'étend également à une utilisation d'un dispositif tel que décrit dans un fixateur externe destiné à maintenir des fiches insérées dans des fragments osseux. Dans les fixateurs externes connus, les fiches sont généralement insérées dans l'os en utilisant un gabarit correspondant à un support de fiches spécifique, ce qui revient à dire que l'insertion des fiches doit s'adapter à la géométrie du fixateur utilisé.

En utilisant des dispositifs d'articulation selon l'invention, on a toute latitude d'insérer chaque fiche dans le fragment osseux à maintenir, selon la configuration de la fracture d'une part, et selon les zones anatomiques favorables à l'insertion, évitant la perforation d'artères, de nerfs ou de ligaments d'autre part. Les fiches ainsi insérées selon des axes très variables sont reliées au fixateur externe, avec interposition d'articulations selon l'invention, qui autorisent leur positionnement précis par rapport au fixateur.

Le dessin annexé représente, à titre d'exemples non limitatifs, des variantes d'exécution de l'objet de la présente invention.

La figure 1 est une vue schématique d'une articulation à rotule conventionnelle et de l'ampleur du mouvement qu'elle autorise.

La figure 2 est une vue similaire à celle de la figure 1, avec une articulation selon l'invention.

La figure 3 est une vue en perspective éclatée des constituants principaux de l'articulation proposée, dans une première forme d'exécution.

La figure 4 est dans sa partie gauche une coupe de l'articulation, selon les axes orthonormés x et y de la figure 3, et dans sa partie droite une vue latérale perpendiculaire à l'axe z, avec arraché partiel.

La figure 5 est dans sa partie gauche une coupe de l'articulation, selon V-V à la figure 4, et dans sa partie droite une vue latérale perpendiculaire à l'axe x.

La figure 6 est une représentation similaire à celle de la figure 5, dans une variante d'exécution proposant une utilisation de l'articulation selon l'invention.

La figure 7 est une vue en perspective éclatée d'une seconde forme d'exécution d'articulation.

Les figures 8 et 9 correspondent, pour cette seconde forme d'exécution, aux figures 4 et 5.

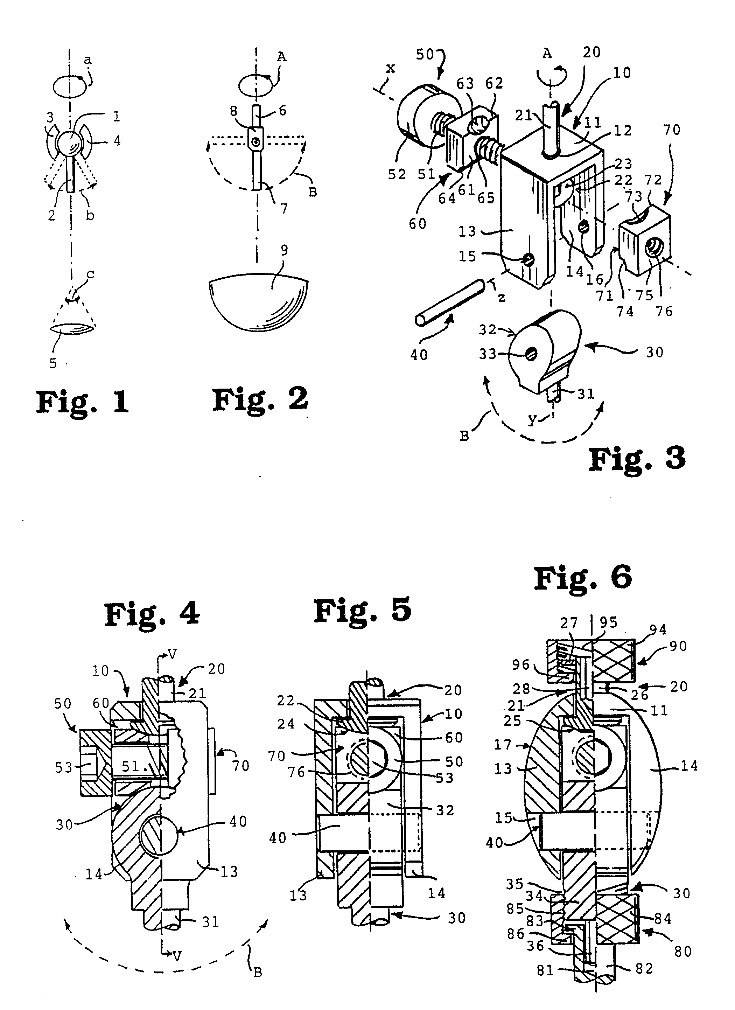

En se référant à la figure 1, on trouve dans la partie supérieure du dessin un assemblage à rotule traditionnel, comportant une partie sphérique 1 prolongée par une tige 2 et une paire de mâchoires 3 et 4. La tige 2 peut être pivotée de 3600 selon la flèche a, mais le basculement selon la flèche b est limité par la présence des mâchoires 3 et 4, de sorte que l'extrémité de la tige 2 ne peut décrire que la calotte sphérique 5 représentée dans la partie inférieure de la figure 1, où l'angle c est généralement limité à 60° environ.

La nouvelle articulation proposée ici et schématisée à la figure 2 comporte une tige 6 et une tige 7 disposées de part et d'autre d'un boîtier 8. En plus de la possibilité de pivoter la tige 6 de 360° selon la flèche A, comme précédemment, on peut balancer la tige 7 selon les flèches B d'un angle de 90° de chaque côté de l'axe vertical, de sorte que l'extrémité de la tige 7 peut décrire l'hémisphère 9 représentée dans la partie inférieure de la figure 2.

Les composants principaux de l'objet de l'invention sont représentés à la vue générale de la figure 3 et comportent un support en double équerre 10, un premier bras d'articulation 20 selon l'axe y d'un système orthonormé, un second bras d'articulation 30 qui peut être aligné sur l'axe du premier et qui est monté pivotant sur un arbre 40, disposé selon l'axe z au dessin. Une vis de serrage 50, disposée selon l'axe x, permet de maintenir deux sabots 60 et 70.

Le support en double équerre 10 est plus précisément constitué par une partie centrale 11, présentant un passage central 12 pour le premier bras d'articulation 20, et par deux ailes 13 et 14, comportant des ouvertures 15 et 16 situées sur un axe commun et dans lesquelles l'arbre 40 vient se fixer. Les faces intérieures de la partie centrale 11 et des ailes 13 et 14 sont planes.

Le premier bras d'articulation 20 comporte un arbre 21 traversant le passage central 12 et une tête 22 présentant un plat intérieur 23 destiné à s'appuyer à l'intérieur de la partie centrale 11 du support 10. Extérieurement, la tête 22 présente une forme particulière, qui sera précisée ultérieurement. L'arbre 21 peut être tourné par rapport au support 10 selon la flèche A.

Le second bras d'articulation 30 comporte un arbre 31, s'étendant perpendiculairement à un corps 32 de forme générale cylindrique présentant un passage central 33. La longueur du cylindre 32 est telle qu'il puisse être introduit avec ébat entre les ailes 13 et 14, et le diamètre du passage central 33 autorise le pivotement du bras d'articulation 30 sur l'arbre 40.

Cet arbre 40 est fixé, par sertissage par exemple, dans les ouvertures 15 et 16 pratiquées à même hauteur dans les ailes 13 et 14. L'arbre 40 traverse librement le passage central 33, de manière à autoriser le balancement, selon la flèche B, du second bras d'articulation 30.

La vis de serrage 50 est destinée à bloquer l'ensemble dans la position désirée. Elle est composée d'une tige filetée 51 et d'une tête cylindrique 52.

Les sabots 60 et 70 sont constitués par des plots de forme générale parallélipipédique, dont les faces en regard 61, 71 comportent sur une arête 62, 72 une creusure 63, 73 correspondant à la forme extérieure de la tête 22 et sur l'arête opposée un dégagement cylindrique 64, 74, destiné à coopérer avec le corps cylindrique 32. Chaque sabot 60, 70 présente encore une ouverture 65, 75 située de préférence au centre du plot. L'ouverture centrale 65 est destinée à laisser libre passage à la tige filetée 51 de la vis de serrage 50, tandis que l'ouverture centrale 75 est munie d'un taraudage 76 destiné à coopérer avec la tige filetée 51, pour assurer le serrage de l'ensemble.

On peut également prévoir de remplacer le taraudage 76 par une ouverture dans laquelle la tige filetée 51 passe librement avant de venir en prise dans un écrou non représenté au dessin.

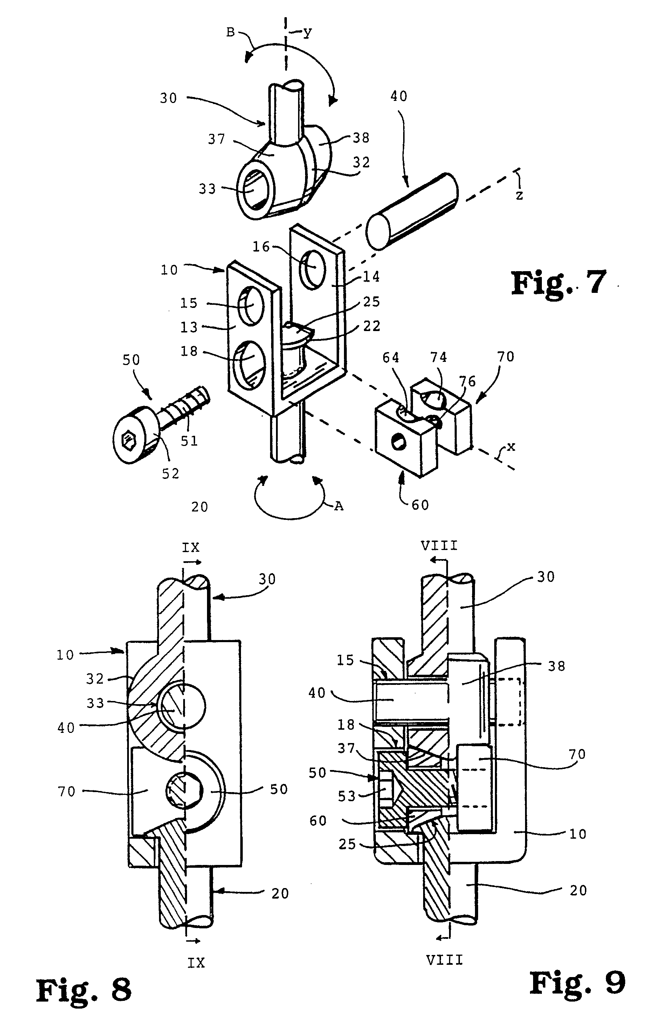

En se référant au dessin des figures 4 et 5, on retrouve le montage des éléments mentionnés précédemment. Bien que les arbres 21 et 31 soient représentés alignés, il est rappelé que le bras d'articulation 30 peut être balancé sur l'axe 40 selon la flèche B, tandis que le bras 20 autorise une orientation de 360° sur l'axe 21.

On remarquera plus particulièrement la forme extérieure de la tête 22 qui est une calotte sphérique 24, destinée à coopérer avec les creusures 63 et 73 des sabots 60 et 70.

On notera également que la tête 52 de la vis 50 comporte une ouverture hexagonale 53 destinée à coopérer avec une clé de serrage 6 pars afin de tourner la tige filetée 51 dans le taraudage correspondant 76 du sabot 70.

Dans la variante proposée à la figure 6 on notera la forme extérieure ellipsoïdale 17 du support en double équerre 10. On remarquera aussi que la tête 22 présente une partie tronconique 25, destinée à coopérer avec des creusures 63,73 tronconiques correspondantes des sabots 60 et 70.

L'articulation de la figure 6 est représentée avec des raccords 80 et 90 assurant le montage de celle-ci dans un ensemble non représenté au dessin.

Le raccord 80 assure la liaison au bras d'articulation 30. A cet effet, l'arbre 34 comporte un filetage 35 et se termine par une tige hexagonale 36, destinée à coopérer avec une ouverture correspondante 81 de la pièce de raccordement 82, qui comporte en outre une collerette 83. Le raccord 80 est fixé par une bague molletée 84 présentant intérieurement un taraudage 85 destiné à coopérer avec le filetage 35, ainsi qu'un épaulement 86 d'appui de la collerette 83.

Le raccord 90 assure de manière similaire la liaison au bras d'articulation 20, dont l'arbre 26 comporte une rondelle 27 et ouverture centrale 28 hexagonale destinée à recevoir une tige de forme correspondante d'une pièce de raccordement, non représentée au dessin. Le raccord 90 est fixé par une bague molletée 94 présentant intérieurement un taraudage 95 destiné à coopérer avec un filetage de la pièce de raccordement, ainsi qu'un épaulement 96 d'appui de la rondelle 27.

Dans la seconde forme d'exécution représentée aux figures 7 à 9, on retrouve la majorité des composants décrits précédemment, portant les mêmes références numériques. Il est simplement à noter que dans les figures 7 à 9, le bras d'articulation 20, destiné à pivoter sur son axe, est représenté vers le bas tandis que le bras d'articulation 30, destiné à pivoter sur l'arbre 40 est représenté en haut des figures.

Cette seconde forme d'exécution se différencie en ce que l'arbre 40 et la vis de serrage 50 sont dans un même plan, au lieu d'être perpendiculaires l'un à l'autre comme précédemment.

A cet effet, l'aile 13 du support en U 10 comporte, en plus de l'ouverture 15 destinée à la fixation de l'arbre 40, une ouverture 18 dimensionnée pour laisser libre passage à la tête 52 de la vis de serrage 50.

Le bras d'articulation 20 comporte une tête 22 présentant une partie tronconique 25, destinée à coopérer avec des creusures correspondantes des sabots 60 et 70.

Le bras d'articulation 30 présente comme précédemment un corps 32 de forme générale cylindrique présentant un passage central 33 assurant l'articulation sur l'arbre 40. Extérieurement le corps 32 comporte deux extrémités tronconiques 37 et 38 destinées à coopérer avec les dégagements 64 et 74 des sabots 60 et 70.

La plupart des pièces mentionnées jusqu'ici sont métalliques et généralement réalisées en acier inoxydable, seul le support en "U" 10 pouvant être en alliage léger. La majorité des angles sont cassés, bien que cela ne soit pas rigoureusement représenté au dessin.

Pour augmenter les qualités d'accrochage de l'articulation, on peut prévoir de réaliser les sabots 60 et 70 avec du carbure de tungstène, ou de traiter les surfaces en contact de la tête de vis 22, des sabots 60 et 70 ainsi que du cylindre 32 en y usinant de légers reliefs (de quelques centièmes de millimètre pour une articulation de l'ordre de quelques centimètres) ou en appliquant un traitement de surface par granulage.

Le montage des éléments formant l'articulation représentée aux figures 3 à 5 se fait en introduisant le premier bras d'articulation 20 dans l'ouverture 12 pratiquée dans la partie centrale du support 10. Puis on fixe le second bras d'articulation 30 entre les ailes 13 et 14 au moyen de l'arbre 40, qui est serti dans les ouvertures et 16 et on enfile les sabots 60 et 70 sur la vis de serrage 50, de part et d'autre de la tête 22 et du corps 32. Une fois l'articulation montée, on peut tourner les bras d'articulation 20 et 30 dans la position désirée avant de serrer la vis 50 dans le sabot 70 afin de maintenir la tête 22 et le corps cylindrique 32 dans la position appropriée.

En position de blocage, le plat intérieur 23 de la tête 22 est maintenu contre la face intérieure de la partie centrale 11 du support 10 tandis que la partie extérieure de la tête 22 repose dans les creusures 63 et 73 des sabots 60 et 70, bloquant ainsi le premier bras d'articulation 20. Les dégagements cylindriques 64 et 74 sont appuyés sur la surface du corps cylindrique 32, bloquant le second bras d'articulation 30.

Il va sans dire que, sans sortir du cadre de l'invention, on peut remplacer le plat intérieur 23 de la tête par une surface sphérique ou tronconique destinée à coopérer avec une creusure ou une protubérance de forme correspondante pratiquée dans la face interne de la partie centrale 11.

Comme on l'a déjà mentionné, l'articulation de la figure 6 est plus particulièrement étudiée pour un fixateur externe de broches insérées dans des parties osseuses, en traumatologie plus particulièrement. Ce dispositif à boitier ellipsoïdal a l'avantage d'éviter les angles et arêtes risquant de gêner le patient et surtout il permet d'insérer librement les fiches dans les zones préférentielles entourant le fragment osseux à maintenir, sans tenir compte des constituants du fixateur lui-même, puisque les fiches peuvent être orientées par rapport à ceux-ci en intercalant des dispositifs d'orientation selon l'invention. Les raccords 80 et 90 sont alors montés aux deux extrémité du dispositif, comme représenté à la figure 6.

On notera que la liaison se fait par un système évitant toute rotation des tiges mises en bout du dispositif décrit puisque la tige hexagonale 36 coopère avec une ouverture correspondante 81 de la pièce à raccorder 82, de même que l'ouverture centrale hexagonale 28 reçoit une pièce à raccorder de forme correspondante.

Il est bien sûr possible de remplacer ces pièces et ouvertures hexagonale par tout autre composant évitant la rotation connu de l'homme de métier, tel qu'un élément comportant des plats, des clavettes, ou autres.

Le montage des éléments formant l'articulation représentée aux figures 7 à 9 se fait en introduisant le premier bras d'articulation 20 dans l'ouverture 12 du support en "U" 10, de telle manière que sa tête 22 repose sur la partie centrale 11. Les sabots 60 et 70 sont mis en place comme représenté à la figure 9. Le corps 32 du second bras d'articulation 30 est introduit entre les ailes 13 et 14 et fixé par insertion de l'arbre 40 dans les ouvertures 15 et 16.

La vis 50 est alors introduite et la tige filetée 51 vient en prise dans le taraudage 76 tandis que la tête 52 s'introduit dans l'ouverture 18 ménagée dans le support en U 10. Le serrage de la vis 50 rapproche les sabots 60 et 70 de telle sorte que :

- les creusures 63 et 73 appuyent sur la partie tronconique 25 de la tête 22, pour éviter la rotation du premier bras d'articulation 20 selon la flèche A; et

- les dégagements 64 et 74 appuyent sur les extrémités tronconiques 37 et 38 du corps cylindrique 32, permettant de bloquer ainsi le second bras d'articulation 30 dans la position angulaire voulue.

Cette seconde forme de construction a pour avantage que les sabots 60 et 70 restent en place lorsque la vis 50 est retirée.

## Revendications

1. Dispositif d'articulation et de blocage relatif de deux pièces allongées, caractérisé par le fait qu'il comporte :

a) un support en U (10) apte à recevoir lesdites pièces,

b) une première (20) pivotable sur un axe,

c) une seconde pièce (30) basculable sur un arbre (40) disposé perpendiculairement audit axe de la première pièce, et

d) des moyens de blocage (60, 70) disposés de part et d'autre desdites première et seconde pièces, sur un arbre de serrage (50) unique.

2. Dispositif d'articulation selon la revendication 1, caractérisé en ce que le support en U (10) comporte une partie centrale (11), présentant un passage central (12) pour la première pièce (20), et deux ailes (13,14), comportant des ouvertures (15,16) situées sur un axe commun aptes à recevoir l'arbre (40).

3. Dispositif d'articulation selon la revendication 2, caractérisé en ce que les faces intérieures de la partie centrale (11) et des ailes (13,14) sont sensiblement planes.

4. Dispositif d'articulation selon la revendication 2 ou 3, caractérisé en ce que l'une des ailes (13,14) comporte une ouverture (18) destinée au passage de l'arbre de serrage (50).

5. Dispositif d'articulation selon la revendication ou 2, caractérisé en ce que la première pièce comporte une tête (22) destinée à coopérer d'une part avec une face intérieure de forme correspondante du support (10) et d'autre part avec les moyens de blocage (60,70).

6. Dispositif d'articulation selon la revendication 5, caractérisé en ce que la tête présente extérieurement une forme de calotte sphérique (24) ou tronconique (25).

7. Dispositif d'articulation selon la revendication ou 6, caractérisé en ce que les moyens de blocage présentent sur leurs faces en regard (61,71) une creusure (63,73) épousant la forme de la partie extérieure de la tête.

8. Dispositif d'articulation selon la revendication 1 ou 2, caractérisé en ce que la seconde pièce présente une partie (32) de forme générale cylindrique disposée perpendiculairement à l'axe de ladite pièce et destinée à coopérer avec les moyens de blocage.

9. Dispositif d'articulation selon la revendication 8, caractérisé en ce que la partie cylindrique (32) possède des extrémités tronconiques (37,38) destinées à coopérer avec les moyens de blocage.

10. Dispositif d'articulation selon la revendication 8 ou 9, caractérisé en ce que la partie cylindrique (32) est apte à être disposée entre les ailes (13,14) du support (10) et comporte un passage central (33) apte à recevoir avec jeu l'arbre (40).

11. Dispositif d'articulation selon la revendication 8 ou 9, caractérisé en ce que les moyens de blocage présentent sur leurs faces en regard (61,71) un dégagement (64,74) épousant la forme extérieure de la seconde pièce.

12. Dispositif d'articulation selon la revendication ou 2, caractérisé en ce que les moyens de blocage comportent un passage (65,75) perpendiculaires aux dites faces en regard apte à recevoir l'arbre de serrage (50).

13. Dispositif d'articulation selon la revendication 12, caractérisé en ce que l'arbre de serrage est constitué par une vis dont la partie filetée (51) est destinée à traverser librement le passage (65) et à venir en prise dans un taraudage (76) pratiqué dans le passage (75).

14. Dispositif d'articulation selon la revendication 12, caractérisé en ce que l'arbre de serrage est constitué par une vis dont la partie filetée (51) est destinée à traverser librement les passages (65,75) et à venir en prise dans un écrou.

15. Dispositif d'articulation selon la revendication 1 ou 2, caractérisé en ce que la première pièce et/ou la seconde pièce sont aptes à coopérer avec un raccord (80,90).

16. Dispositif d'articulation selon la revendication 15, caractérisé en ce que le raccord comporte des moyens de fixation à ladite pièce ainsi que des moyens évitant la rotation relative entre un élément à raccorder et le dispositif.

17. Utilisation d'un dispositif d'articulation et de blocage relatif de deux pièces dans un fixateur externe orthopédique composé de fiches insérées dans des fragments osseux et d'une armature externe comportant des tiges de liaison, caractérisée par le fait que sur au moins une desdites fiches et/ou au moins une desdites tiges de liaison on dispose un dispositif d'articulation et de blocage relatif de deux pièces allongées comportant :

a) un support en U (10) apte à recevoir lesdites pièces,

b) une première pièce (20) pivotable sur son axe,

c) une seconde pièce (30) basculable sur un arbre (40) disposé perpendiculairement audit axe de la première pièce, et

d) des moyens de blocage (60, 70) disposés de part et d'autre desdites première et seconde pièces, sur un arbre de serrage (50) unique.

18. Utilisation d'un dispositif d'articulation selon la revendication 17, selon laquelle ladite tige de liaison est confondue avec la première ou la seconde pièce.

19. Utilisation d'un dispositif d'articulation selon la revendication 17 ou 18, selon laquelle ladite ladite tige de liaison est reliée avec la première ou la seconde pièce au moyen d'un raccord.

**Fig. 1**   **Fig. 2**

**Fig. 3**

**Fig. 6**

**Fig. 4**   **Fig. 5**

**Fig. 7**

**Fig. 8**

**Fig. 9**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-1 123 300  (BERGON)<br>* En entier * | 1,2,3,4 | F 16 C 11/10<br>A 61 B 17/60 |
| | — — — | | |
| A | | 5,12,13,<br>14,15 | |
| X | CH-A-3 034 53  (HOFFMANN)<br>* En entier * | 1,4,15,17 | |
| | — — — | | |
| A | | 2,3,18 | |
| A | FR-A-9 273 70  (CARROUEE)<br>* Page 2, lignes 21-51; figures 5,6 * | 1,8,10,11,<br>12,13 | |
| | — — — | | |
| A | US-A-2 887 329  (BLAKELY)<br>* En entier * | 1,15 | |
| | — — — | | |
| A | CH-A-2 450 92  (PAILLARD)<br>* En entier * | 1 | |
| | — — — | | |
| A | DE-C-8 077 20  (FLAD)<br>* En entier * | 1 | |
| | — — — | | **DOMAINES TECHNIQUES RECHERCHES (Int. Cl.5)** |
| A | FR-A-2 346 594  (PFLIEGER) | | F 16 C<br>F 16 M<br>A 61 B<br>A 61 F |
| | — — — — — | | |

Le présent rapport de recherche a été établi pour toutes les revendications

| Lieu de la recherche | Date d'achèvement de la recherche | Examinateur |
|---|---|---|
| La Haye | 10 décembre 90 | ORTHLIEB CH.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

 

& : membre de la même famille, document correspondant